# EUROPEAN PATENT SPECIFICATION

(11) **EP 1 106 692 B1**
(45) Date of publication and mention of the grant of the patent: **15.06.2005**
(21) Application number: 99935124.0
(22) Date of filing: 10.08.1999
(51) Int. Cl.: C12N 15/40, C12N 7/04, C12N 5/06, A61K 48/00

(54) **RNA VIRUS VECTOR HAVING CONTACT INFILTRATION CAPABILITY**
RNS VIRUS VEKTOR MIT DER FÄHIGKEIT ZUR KONTAKTINFILTRATION
VECTEUR VIRAL RIBONUCLEOTIDIQUE AYANT LA CAPACITE DE S'INFILTRER PAR CONTACT

(30) Priority: 11.08.1998 JP 22739898
(43) Date of publication of application: 13.06.2001
(73) Proprietor: Dnavec Research Inc., Tsukuba-shi, Ibaraki 305-0856 (JP)
(72) Inventor: ASAKAWA, Makoto 319, Shionogi-Kanzakigawa-Ryo, Toyonaka-shi Osaka 561-0825 (JP); HASEGAWA, Mamoru DNAVEC Research Inc., Tsukuba-shi Ibaraki 305-0856 (JP)
(74) Representative: Becker Kurig Straus
(86) International application number: PCT/JP1999/004333
(87) International publication number: WO 2000/009700

(56) References cited:
- EP-A- 0 864 645
- WO-A1-97/16538
- CATHOMEN TONI ET AL: "A matrix-less measles virus is infectious and elicits extensive cell fusion: Consequences for propagation in the brain." EMBO (EUROPEAN MOLECULAR BIOLOGY ORGANIZATION) JOURNAL, vol. 17, no. 14, 15 July 1998 (1998-07-15), pages 3899-3908, XP002180605 ISSN: 0261-4189
- RADECKE F ET AL: "RESCUE OF MEASLES VIRUSES FROM CLONED DNA" EMBO JOURNAL, OXFORD UNIVERSITY PRESS, SURREY, GB, vol. 14, no. 23, 1 December 1995 (1995-12-01), pages 5773-5784, XP002022952 ISSN: 0261-4189
- HASAN MOHAMMAD K ET AL: "Creation of an infectious recombinant Sendai virus expressing the firefly luciferase gene from the 3' proximal first locus." JOURNAL OF GENERAL VIROLOGY, vol. 78, no. 11, November 1997 (1997-11), pages 2813-2820, XP000886572 ISSN: 0022-1317
- GENEVIEVE MOTTET ET AL.: 'A Sendai Virus Vector Leading to the Efficient Expression of Mutant M Proteins Interfering with Virus Particle Budding' VIROLOGY vol. 221, no. 0362, 1996, pages 159 - 171, XP002925173
- TORU KONDO ET AL.: 'Temperature-sensitive Phenotype of a Mutant Sendai Virus Strain is Caused by its Insufficient Accumulation of the M Protein' THE JOURNAL OF BIOLOGICAL CHEMISTRY, vol. 268, no. 29, 15 October 1993, pages 21924 - 21930, XP002925174

## Description

### Technical Field

The present invention relates to a virus vector that can be used for gene therapy, and more specifically, to an inactivated negative strand RNA ((-)RNA) viral vector.

### Background Art

In gene therapy for humans and animals, effectiveness and safety are very important. Particularly, in therapy using a "virus vector" which is obtained by viral gene recombination, treatment must be carried out with extreme care, when it is hard to discriminate the possibilities of nonspecific chromosomal integration, and leakage of recombinant or pathogenic viruses to nature, uncontrolled expression of an introduced gene even if the treatment is effective.

Virus vectors are mainly used for introducing a gene of interest into target cells by utilizing viral infectivity. Recombinant virus vectors, which have been genetically engineered to carry a gene insert(s), contain viral envelope proteins on the surface. Since the envelope proteins retain viral infectivity, it is possible to introduce its endogeneous recombinant gene inserts into cells. These vectors can be utilized for not only gene therapy but also production of cells expressing a gene of interest and creation of transgenic animals.

Virus vectors are classified into three: retrovirus vectors, DNA virus vectors, and RNA virus vectors. Among them, RNA virus vectors which are not integrated into chromosomes, are advantageous in terms of safety. According to this technical background, virus vectors derived from non-segmented (-) RNA virus such as Sendai virus have already been provided (D. Yu et al., Genes to Cells, 2:457-466, 1997; M. Hasan et al., J. Gen. Virol. 78:2813-2820, 1997). The present inventors have developed a (-)RNA virus vector that has infectivity and autonomous RNA replication ability without disseminative capability (WO 97/16538).

### Disclosure of the Invention

An objective of the present invention is to provide the use of a Sendai virus vector for gene therapy.

. Using nucleic acid of Sendai virus, a representative of (-)RNA viruses, which is supposed to be the most useful vector for industrial applications in terms of safety and convenience, the present inventors succeeded in obtaining various defective mutants, which was experimentally reconstituted using known methods. As a result, the inventors found that the complexes reconstituted by the mutants carrying a defect in M gene were capable of forming plaques, which were smaller than those formed by wild type viruses, but incapable of propagating in embryonated chicken eggs. Thus, the inventors confirmed that the mutants are capable of cell infection, autonomous RNA replication, and contact infiltration, but incapable of dissemination. Furthermore, the plaques formed by a defective mutant carrying defect in the M gene were stained with anti-Sendai virus antibody, but not with anti-M monoclonal antibody. From this result the inventors confirmed that plaques formed by the M mutants are lacking complete M protein, thereby accomplishing the present invention.

Thus, the present invention provides the use of a Sendai virus RNA comprising genes involved in contact infiltration and autonomous RNA replication, but no or inactivated genes involved in dissemination for transferring a foreign gene from a cell to another cell through contact infiltration.

The present invention also provides the use of a cell comprising the above-described RNA, which is capable of allowing the RNA to replicate therein and transmitting said RNA to another cell through contact infiltration.

Furthermore, the present invention provides the use of a Sendai virus complex capable of cell infection, contact infiltration, and autonomous RNA replication but incapable of dissemination.

Moreover, the present invention provides the use of a DNA comprising a template DNA for transcribing the above-described RNA *in vitro* or in a cell for transferring a foreign gene from a cell to another cell through contact infiltration.

Herein, "cell infectivity" of a virus vector means "an ability of a viral vector capable of cell adhesion and membrane fusion to introduce its endogeneous nucleic acids into cells." "Disseminative capability" means "an ability of a nucleic acid introduced into a cell artificially or by infection to form infective particles or comparable complexes following replication of said nucleic acid in the cell, and then be transmitted into another cell." "Contact infiltration capability" means "an ability of a cell carrying a viral vector gene to transfer the gene to another cell through contact."

A viral vector capable of contact infiltration but incapable of dissemination can overcome a critical demerit of existing virus vectors, which are incapable of dissemination and contact infiltration. In other words, when cells are infected with a virus vector to express a gene insert in the virus vector, the existing virus vectors are only able to express a protein in cells directly infected. This is not a problem when cell culture systems are used, in which the cells grow in a layer and can be directly infected with viral vectors. However, when *in vivo* cells that exist three-dimensionally in multi-layers are infected with viral vectors, the region and the number of cells directly infected by the existing vectors are limited. For example, it is extremely difficult to directly infect all tumor-forming cells at a tumor site with the viral vectors.

By contrast, even if Sendai virus vectors capable of contact infiltration of the present invention are used to infect tumor cells at a tumor site and only a part of the cells are directly infected, noninfected cells adjacent to the infected cells can be infected with the vectors through contact infiltration, resulting in infecting all the tumor cells. Since the vector is not disseminative, there is no possibility that the vector infects other cells in the body through blood stream.

Thus, the virus vector has such novel features that it infects all the cells in a restricted region or a particular organ and does not infect the other part of the body.

Furthermore, cells carrying the virus vector of the present invention capable of cell infection, autonomous RNA replication, and contact infiltration, but incapable of disseminative, can be utilized for cell transplantation. Since a cell carrying a virus vector capable of autonomous replication and contact infiltration can transfer the vector to another cell, it is possible to transplant the cell carrying the vector at the minimal dose to a diseased site, thereby treating there.

It has been reported that the formation of infective particles of Measles virus having a defect in theM gene is inhibited, and cell fusion is promoted (T. Cathomen et al., EMBO J., 17:3899-3908, 1998). However, the possibility to utilize the virus as a vector has not been suggested.

A source of a (-)RNA virus includes Sendai virus, Newcastle disease virus, Mumps virus, Measles virus, Respiratory syncytial virus, rinderpest virus, and distemper virus of the Paramyxoviridae; influenza virus of the Orthomyxoviridae, and Vesicular S, and Rabies virus of the rhabdoviridae. Incomplete viruses such as DI particle (J. Virol. 68:8413-8417, 1994) and synthetic oligonucleotides can also be used.

.A recombinant (-) RNA virus derived from any viruses described above can also be used as a vector source. For example, the recombinant (-)RNA viruses may be those with the gene for antigenicity inactivated or those with some genes altered to improve efficiency of RNA transcription or replication

. An RNA used in the present invention can be obtained by transcribing an altered cDNA derived from or recombinant Sendai virus *in vitro* or in cells. In the obtained RNA, at least one gene involved in disseminative capability of the original virus must be deleted or inactivated, but gene involved in autonomous replication and contact infiltration should not be deleted or inactivated. In addition, an RNA molecule with artificial sequences, which are prepared by transcribing is *in vitro* or in cells a DNA obtained by inserting genes for autonomous replication into a cDNA containing the structure of both ends of a virus genome such as DI molecules can also be used.

In Sendai virus, "genes involved in autonomous replication" are either of NP, P/C, or L genes, and "gened involved in transmission" are either of M, F, or HN genes. When only the M gene is deleted or inactivated, "disseminative capability" will be lost, but "contact infiltration ability" will remain because virion or virus-like particles capable of releasing the complex containing genomic RNA extracellularly are not formed. Therefore, for example, an RNA of Sendai virus Z strain deficient in only the M gene and a ribonucleoprotein (RNP) comprising said RNA are suitably used in the present invention. The genes are not necessary to be the same as the original viral sequences. They can be mutated or substituted by the corresponding gene of other viruses as long as the resulting gene has transcription and replication activities comparable to or higher than those of the wild type.

An RNA used in the present invention may have a foreign gene inserted to an appropriate site. To express a desired protein, a foreign gene encoding the protein is inserted. In the case of Sendai virus RNA (GenBank accession No. M30202), a sequence with a multiple of six bases between the R1 and R2 sequences (J. Virol., Vol. 67, No. 8, 1993, 4822-4830). The consensus sequences of R1 and R2 are 5'-AGGGWBAAWGD-3' and 5'-DTAAGAAAAA-3', respectively. The expression level of the inserted foreign gene can be regulated by the inserted position or the RNA nucleotide sequences flanking the inserted gene. For example, it is known about Sendai virus RNA that the expression level of the inserted gene is higher as the insert position is nearer to the NP gene.

A complex used in the present invention comprises a virus structure without the above-described RNA and nucleic acid. "A virus structure without nucleic acid" is, for example, a virus from which only RNA is removed and complements infectivity and autonomous replication ability of the RNA used in the present invention, not complement ability to form particles that release the complex extracellularly and move freely, in other words, not complement the disseminative capability. In Sendai virus, a complex of the RNA in which only the M gene is deleted and the virus structure from which the RNA and M protein are removed (RNP), has infectivity and autonomous replication ability, but not disseminative capability. The complex can contain any other components as long as they do not exhibit disseminative capability. For example, molecules that binds to a specific cell, such as adhesion molecules, ligands, or receptors, can be included on the envelope surface.

Moreover, a kit comprises a) the above-described RNA of the present invention, a cRNA of said RNA, or a unit that is capable of biosyntheeizing said RNA or said cRNA, and b) a group of enzymes required for replication of said RNA or said cRNA, or a unit that is capable of biosynthesizing said enzymes. The complex used in the present invention, which is capable of cell infection, autonomous RNA replication, and contact infiltration, but incabable of dissemination, can be produced by introducing units of a) and b) into an appropriate host. Producing the complex used in the present invention means reconstituting an RNP having autonomous replication ability. When RNA derived from Sendai virus is used for a), b) is preferably all of NP, P/C, and L proteins derived from Sendai virus or a unit capable of biosynthesizing said proteins.

A host is not limited as long as it allows virus RNA used to be expressed therein. When RNA derived from Sendai virus is used, any cells susceptible to the infection with Sendai virus can be used, including cultured mammalian cells, cultured avian cells, and embryonated chicken eggs. Examples of cultured cells are LLCMK2, MDCK, MDBK, CV-1, Hela, HepG2, P19, F9, CHO, PC12, 293 cell, BAF3, Jerkat, human PBMC, MT-4, Molt-4, NIH3T3, L929, chicken embryo fibroblast, etc.

The present inventors have shown that Sendai virus CDNA reconstitutes particles more efficiently when cDNA to be introduced into cells is in a linear form than in a circular form, and that transcription of a (+)RNA in cells more successfully forms particles than that of a (-)RNA (A. Rato et al., Genes to Cells, 1: 569-579, 1996). Although these conditions may not always be applicable to the reconstitution of all other (-)RNA viruses, it is possible to determine the appropriate conditions for the reconstitution of other (-)RNA viruses according to the description of the present specification and based on conventional technology.

The produced complex can be recovered by the standard methods from the host, for example, cultured cells or embryonated chicken eggs.

A foreign gene encoded by the RNA constituting a complex can be expressed by infecting cells with the complex. Namely, the foreign gene can be expressed *in situ* by inoculating the complex or a cell carrying it into a host such as embryonated chicken eggs or appropriate tissues of an animal body. Alternatively, cells recovered from a living body are transformed so that the complex can autonomously replicate, and the transformed cells are returned to the living body to allow the cells to express the foreign gene.

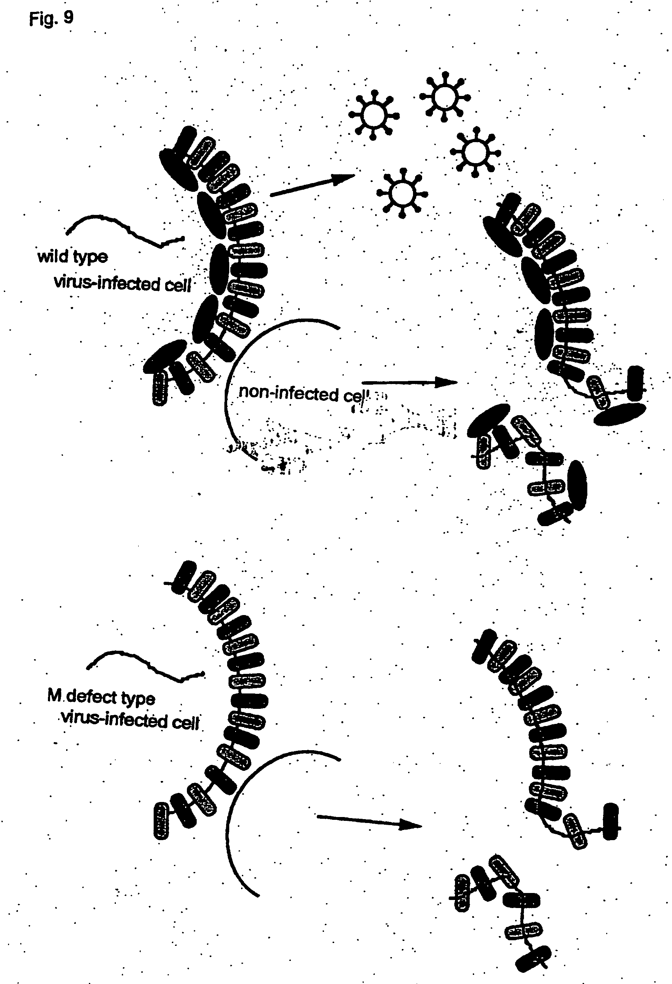

Figure 9 shows the processes of infecting target cells with a Sendai virus complex in which the M gene is deleted or inactivated and infiltrating the RNA used in the present invention into surrounding non-infected cells through contact infiltration.

### Brief Description of the Drawings

Figure 1 schematically shows the structure of the plasmid pUC18/T7(+)HVJRz.

Figure 2 schematically shows the region carrying the M gene in Sendai virus cDNA and procedure for subcloning or mutating said region. In the figure, E stands for transcription termination sequence; I, intervening sequence; and S, transcription initiation sequence. Boxes indicate the restriction sites used for the plasmid construction.

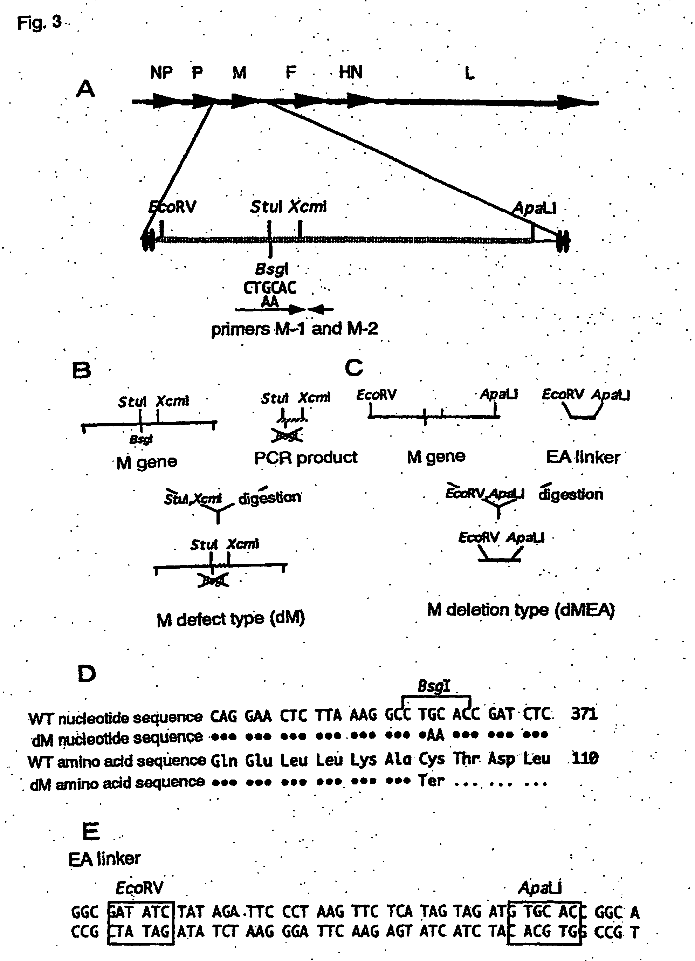

Figure 3 schematically shows procedures for introducing mutation to the M gene region of Sendai virus cDNA. Upper panel of A shows the structure of Sendai virus genome with each position of the NP, P (P/C), M, F, HN, and L genes. Lower panel shows the structure of the M gene, restriction sites, and positions of primers M1 and M2 used for the construction of the M defect type. B and C schematically show the construction of the M defect type and the M deletion type, respectively. Crossed out restriction sites indicate that they are mutated to be indigestible by the enzymes. D shows comparison between the M defect and wild type M genes in terms of both nucleotide and amino acid sequences. Dots in the M defect sequences mean that bases or amino acids are identical to the corresponding wild -type sequences. [Ter] indicates a termination codon. Numerals indicate the base/amino acid position in the M gene sequence.

Figure 4 schematically shows procedure for subcloning the M gene region of Sendai virus cDNA. Crossed out restriction sites indicate that they are mutated to be indigestible by the enzymes.

Figure 5 schematically shows the construction of the M deletion type cDNA by mutating the subcloned Sendai virus M gene region and replacing the full-length M gene region of Sendai virus cDNA with the mutated one. Crossed out restriction sites indicate that they are mutated to be indigestible by the enzymes.

Figure 6 schematically shows the construction of the M defect type cDNA by mutating the subcloned Sendai virus M gene region, and replacing the full-length M gene region of Sendai virus cDNA with the mutated one. Crossed out restriction sites indicate that they are mutated to be indigestible by the enzymes.

Figure 7 shows plaques of Sendai virus cDNA of wild type (WT1 and WT2); M defect type (dM); and M deletion type (dMEA2-1 and dMEA2-2).

Figure 8 shows plaques of Sendai virus cDNA of wild type (WT); M defect type (dM); and M deletion type (dMEA), which were stained with anti-Sendai virus antibody.

. Figure 9 illustrates the process of contact infiltration by Sendai virus genome of wild type genome (upper panel) and of the mutant with mutated or no M gene (lower panel). The wild type forms infective virus particles and causes contact infiltration, while the mutant is not able to form infective particles but introduces its genome into non-infected surrounding cells only by contact infiltration.

### Best Mode for Carrying out the Invention

The present invention will be illustrated in detail below with reference to Examples, but is not to be construed as being limited thereto.

### Example 1. Subcloning of the M gene of Sendai virus

All of the following ligation, blunting, and dephosphorylation were performed using the Takara Ligation Kit Ver.2 (Takara Shuzo, Kyoto, Japan), the Takara Blunting Kit (Takara Shuzo), and CIAP (Takara Shuzo), respectively, according to the manufacture's protocols attached to the products. The nucleotide sequences were determined by Sanger's method (F. Sanger, Science, 214: 1205-1210, 1981).

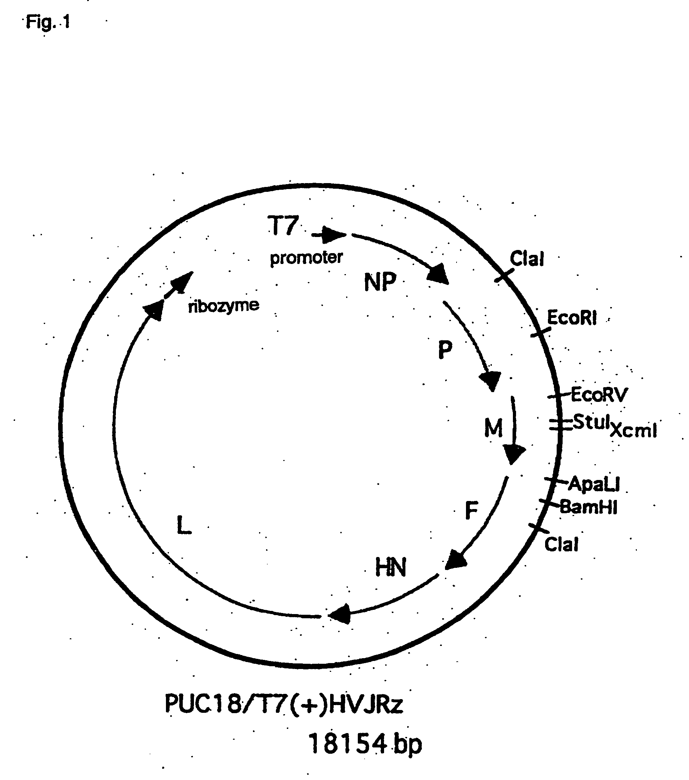

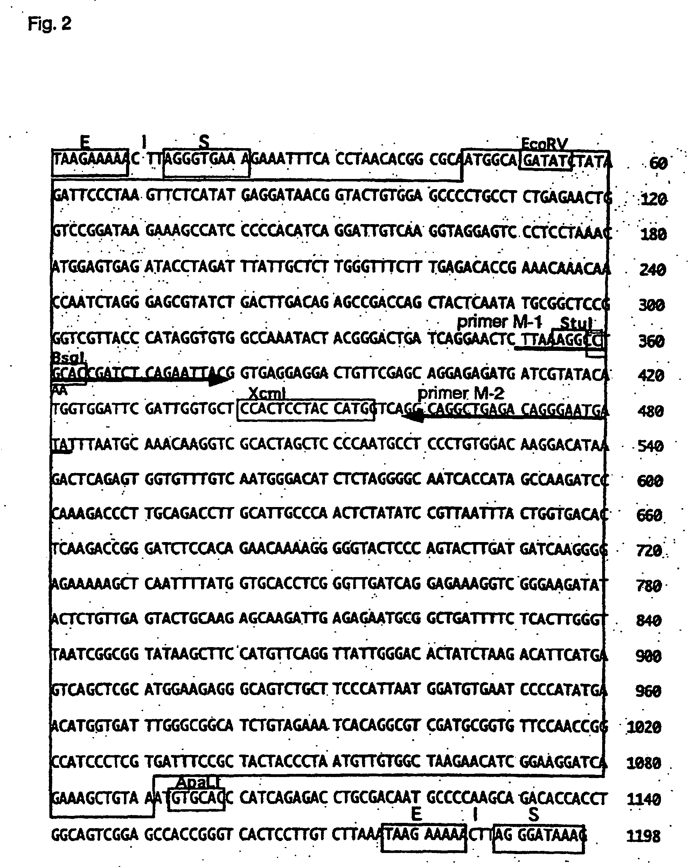

Complementary DNA in which the M gene was deleted was constructed as follows, using wild type cDNA, pUC18/T7(+)HVJRz (A. Kato et al., Genes to Cells, 1: 569-579, 1996) (Figure 1). The DNA sequences of the M gene and its vicinity are shown in Figure 2, and the construction scheme is shown in Figure 3.

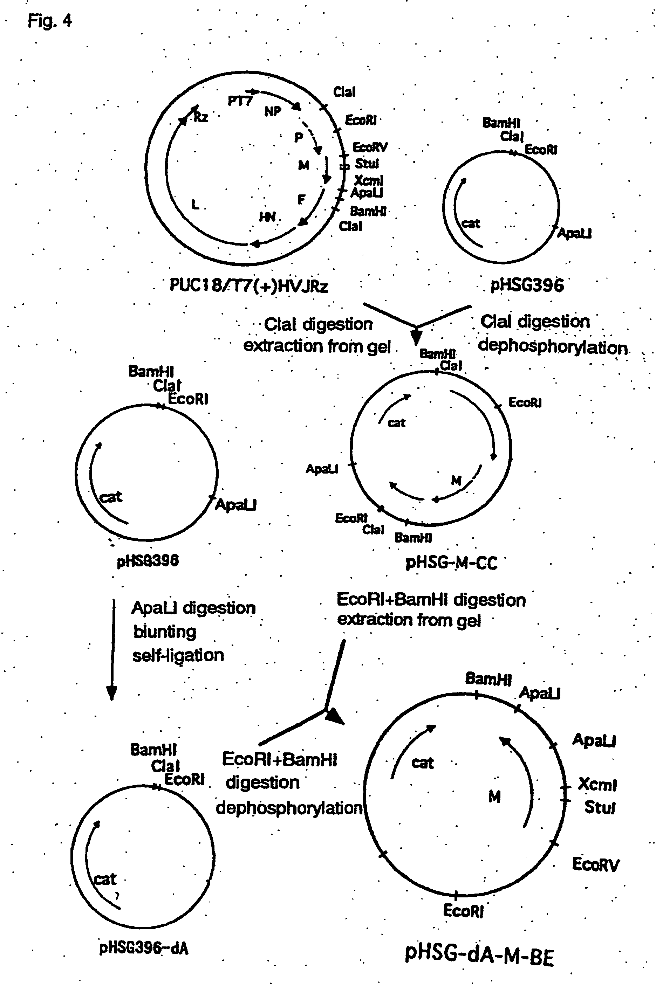

First, the ClaI fragment containing the M gene was excised from pUC18/T7(+)HVJRz and inserted into the ClaI site of pHSG396 (Takara Shuzo) to obtain pHSG-M-CC (Figure 4). Separately, pHSG396 was digested with ApaLI, blunted, and self-ligated to obtain pHSG396-dA that lacks the ApaLI site (Figure 4). The EcoRI-BamHI fragment of pHSG-M-CC carrying the M gene was inserted into dephosphorylated EcoRI-BamHI-digested pHSG396-dA to obtain pHSG-dA-M-BE (Figure 4).

### Example 2. Construction of M deletion type vector

Forty nine-mer synthetic oligonucleotides, 5'-ggcgatatctatagattccctaagttctcatagtagatgtgcaccggca-3' (EA linker F) (SEQ ID NO: 1) and 5'-tgccggtgcacatctactatgagaacttagggaatctatagatatcgcc-3' (EA linker R) (SEQ ID NO: 2), were annealed and inserted into pCR2.1 (Invitrogen) to generate pCR-EAL. The nucleotide sequence of the insert was verified to be as designed.

The pCR-EAL was digested with EcoRV and ApaLI, and the linker fragment was excised. Separately, the pHSG-dA-M-BE was digested with EcoRV and ApaLI and the above linker fragment was inserted therein to obtain pHSG-dA-dM-EA-EB.

. The pHSG396 was digested with EcoRI, blunted, and self-ligated to obtain a plasmid lacking the EcoRI site, which was further digested with BamHI, blunted, and self-ligated to obtain pHSG-dE-dB, a plasmid lacking both EcoRI and BamHI sites.

The ClaI fragment carrying the M gene was inserted into the ClaI site of pHSG-dE-dB to generate pHSG-dE-dB-M-CC. The EcoRI-BamHI fragment excised from pHSG-dA-dM-EA-EB was inserted into the EcoRI-BamHI-digested pHSG-dE-dB-M-CC to obtain pHSG-dE-dB-dM-CC.

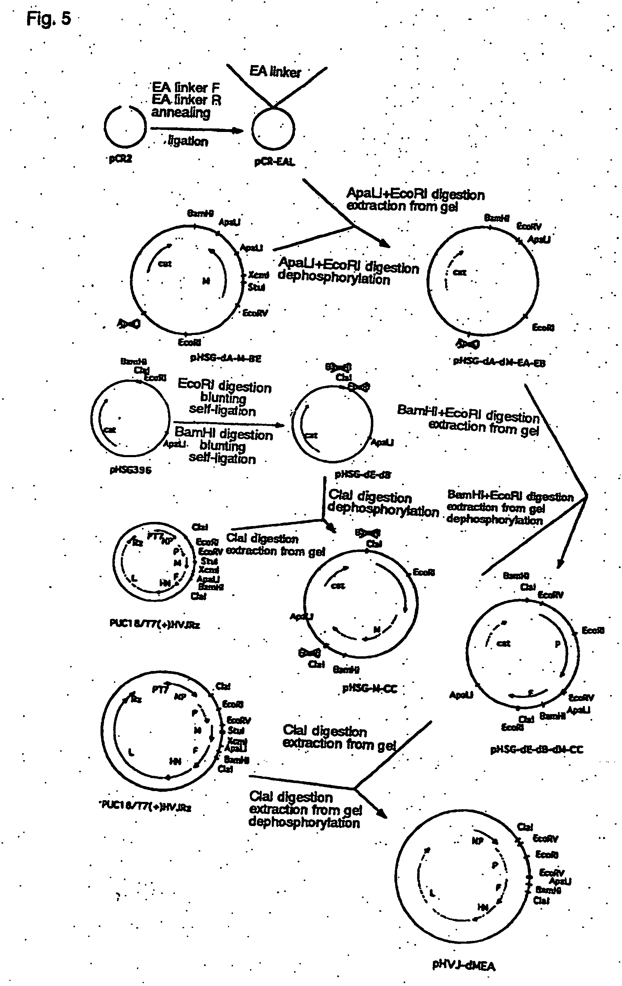

The ClaI fragment of pHSG-dE-dB-dM-CC was inserted into the ClaI site of the pUC18/T7(+)HVJRz to obtain pHVJ-dMEA. This construction scheme is shown in Figure 5.

### Example 3. Construction of M defect type vector

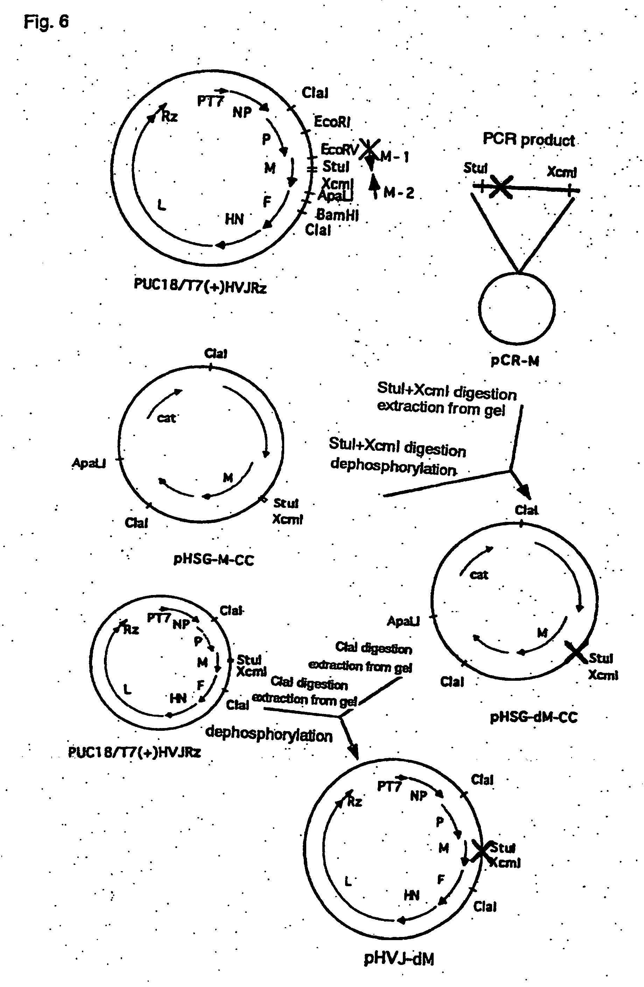

PCR was performed using pUC18/T7(+)HVJRz as a template with oligonucleotide primers, M-1 (5'-ttaaaggcctaaaccgatctcagaattacg-3')(SEQ ID NO: 3), and M-2 (5'-tatcattccctgtctcagcctgcc-3')(SEQ ID NO: 4). The primers were designed to introduce a mutation at the BsgI site of theM gene so that a stop codon appears in frame of the M gene. The PCR products were subcloned into pCR2.1 to obtain pCR-M (Figure 6).

Following verification of the nucleotide sequence of pCR-M, the StuI-XcmI fragment of pCR-M was recovered from the gel and inserted into the StuI-XcmI-cut pHSG-M-CC. The resulting plasmid was named as pHSG-dM-CC. The ClaI fragment of pHSG-dM-CC was inserted into the ClaI site of pUC18/T7 (+) HVJRz to obtain pHVJ-dM. This construction scheme is shown in Figure 6.

### Example 4. Reconstitution of virions from M deletion type and M defect type cDNAs

Reconstitution of virions from cDNA of wild type (WT), M deletion type (dMEA), and M defect type (dM) was performed as follows.

Cultured LLCMK2 cells (a simian kidney cell line) were detached from a dish by treatment with trypsin, and 2 x 10⁶ cells were plated in a plastic dish of 10 cm diameter and incubated in MEM medium (Gibco BRL) supplemented with 10% FBS (Gibco BRL) at 37°C for 24 hours in 5% CO₂. The medium was then removed, and the cells were washed once with PBS. Five hundred µl of a solution of vTF7-3, a recombinant vaccinia virus vector capable of expressing T7 phage RNA polymerase (T.R. Fuerst et al., Proc. Natl. Acad. Sci. USA, 83:8122-8126, 1986), which was diluted with PBS to 8 x 10⁶ pfu/ml to obtain MOI (multiplicity of infection) of 2, was added dropwise to the dish. The cells were infected for 1 hour with agitating the dish every 15 minutes to spread the virus solution over the whole dish.

Separately, a medium containing a cDNA solution was prepared as follows. First, 500 µl Opti-MEM (Gibco BRL) without FCS (fetal calf serum) was added into a polystyrene tube, and then any of pUC18/T7 (+) HVJRz, pHVJ-dMEA or pHVJ-dM, all plasmids originating from Sendai virus cDNA (20 µg), pGEM-NP (5 µg), pGEM-P (2.5 µg), and pGEM-L (5 µg) were added. Fifty µl SuperFect (QIAGEN) was further added, and the mixture was allowed to stand at room temperature for 20 minutes. Ten ml of Opti-MEM was then added. Subsequently, Rifampicin and Cytosine arabinoside (Ara C) were added at the final concentration of 100 µg/ml and 40 µg/ml, respectively.

After 1-hour infection, the virus solution was removed from the above dish containing LLCMK2 cells, the medium containing the above cDNA solution was added to the culture by decantation, and the cells were incubated at 37°C for 48 hours in 5% CO₂. The cells were scraped using a cell scraper without removing the medium, and the cells and the medium were transferred together to a 15-ml centrifuge tube. After centrifugation at 1200 rpm for 5 minutes, the supernatant was removed, and the precipitated cell was resuspended in 200 µl of PBS.

### Example 5. Inoculation of cell suspensions to embryonated chicken eggs and HA assay

To evaluate virus reconstitution, an assay using embryonated chicken eggs was performed. The cells suspended in 200 µl of PBS obtained in Example 4 were adjusted to 1 x 10⁷ cells/ml and sequentially diluted with PBS to obtain cell suspensions of 1 x 10⁶ and 1 x 10⁵ cells/ml. One hundred µl of each suspension was inoculated to a 10-day-old embryonated chicken egg through the air chamber side using a 24G needle.

The chicken eggs were incubated at 35.5°C for 3 days with rotating. Then, the eggshell at the air chamber was cut out, and urine was collected using a 10-ml syringe with 18G needle.

The collected urine was tested by HA assay to evaluate virus propagation in the chicken eggs. HA assay was performed as follows.

Fifty µl of PBS was dispensed into each well of from the second to the 12th lines of a 96-well round bottom plate. One hundred µ l of the urine sample was added to the first line. Fifty µl of the sample in the first line was mixed with PBS in the second line to make a 1:2 dilution. Fifty µl of the 1:2 dilution was mixed with PBS in the third line to make a 1:4 dilution. By repeating the procedure, a series of two-fold dilutions were made.

Fifty µl of 1% chicken preserved blood (Cosmo Bio) diluted with PBS was added to all the lines, and the plate was kept at 4°C for 1 hour. Erythrocyte aggregation was examined with the naked eye and the highest dilution that results in the aggregation was shown as HA activity (Table 1).

**Table 1**

| Virus cDNA | HA activity |
|---|---|
| WT (wild type) | > 16 |
| dM (defect) | < 2 |
| dMEA2-1 (deletion) | < 2 |
| dMEA2-2 (deletion) | < 2 |

Virus cDNAs used were of viruses, wild type (pUC18/T7(+)HVJRz; WT), the M defect type (pHVJ-dM; dM), and the M deletion type (pHVJ-dMEA; dMEA). For the M deletion type, the results of two experiments were shown (dMEA2-1, and dMEA2-2). The wild type virus showed an activity higher than 16, whereas the other samples did not show any significant activity. The result indicates that the Sendai virus cDNA in which the M gene is deleted or is defective did not produce a virus with disseminative capability in the standard reconstitution experiment.

### Example 6. Plaque formation

CV-1 cells were plated at 5 x 10⁵ cells/well into a 6-well plate (Corning, microplate 6-well) and cultured overnight.

LLCMK2 cells obtained in Example 4 were diluted with PBS to 1 x 10⁶ cells/ml, and 100 µl of the suspension was dispensed into 1.5 ml microtubes (Eppendorf). A portion of the dispensed suspensions was subjected to three repeats of freeze-thawing by freezing at -80°C for 10 minutes and thawing in water at room temperature for 3 minutes. Trypsin was added to all the cell suspensions at the final concentration of 0.75 µg/ml and the mixtures were incubated at 37°C for 30 minutes.

The media were removed from the plates of CV-1 cell culture, and the cells were washed once with PBS, then overlaid with 100 µ l of the above diluted cell suspension treated with trypsin. The mixtures were incubated for 1 hour with agitating every 15 minutes so as to spread the suspension over the whole plate. The plates were overlaid with 3 ml of 1 x MEM containing 1% agar, supplemented with 0.1% BSA, Rifampicin 100 µg/ml, Ara C 40 µg/ml, and trypsin 0.75 µg/ml, that was preheated to approximately 45°C. After the agar solidified, the plates were inverted and incubated at 37°C for 5 days in 5% CO₂.

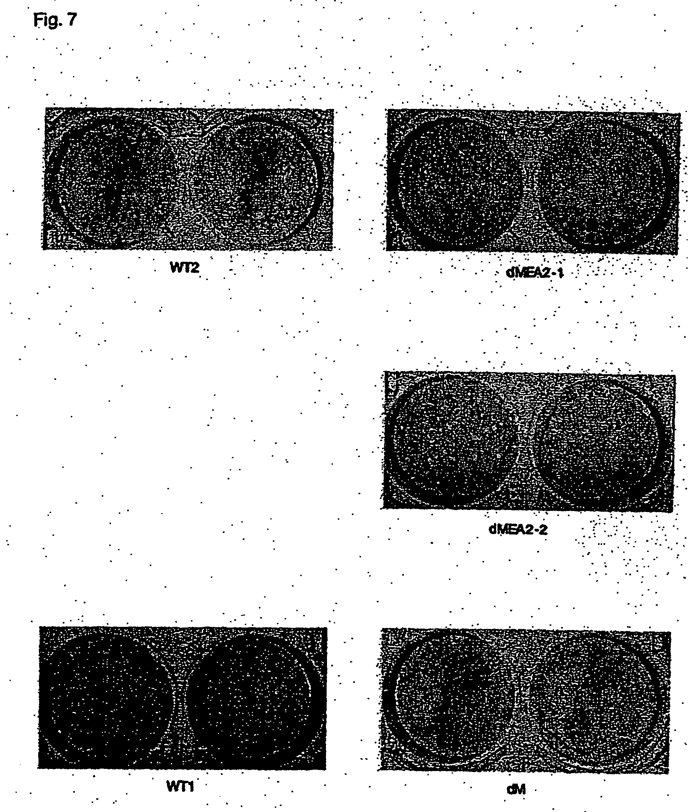

Then, 1 ml of a fixative (ethanol:acetic acid = 5:1) was added onto the agar, and the plates were allowed to stand at room temperature for 90 minutes. The agar was then removed, and 200 µl of Amido Black solution (0.5% Amido Black, ethanol:acetic acid:water = 45:10:45) was added. Immediately thereafter, the cells were washed with water and stained to count the plaque number and evaluate the shapes of plaques. The results were shown in Table 2 and Figure 7.

**Table 2**

| Reconstitution efficiency of Sendai virus deficient or defective in the M gene | | | |
|---|---|---|---|
| M gene | Number of plaques | | |
| | freeze-thawed | | not frozen |
| | Run 1 | Run 2 | |
| wild type | 38 | 22 | 14 |
| M defect type (dM) | 2 | 4 | 2 |
| M deletion type (dMEA2-1) | 27 | 26 | 60 |
| M deletion type (dMEA2-2) | 32 | 38 | 42 |

Virus cDNAs used are of wild type (pUC18/T7(+)HVJRz), the M defect type (pHVJ-dM), and the M deletion type (pHVJ-dMEA). For the M deletion type, the results of two experiments (dMEA2-1 and dMEA2-2) are shows. The number of plaques was counted for both freeze-thawed and not frozen cell suspensions. The plaque number reflects the number of reconstituted viruses in 1 x 10⁵ transfected LLCMK2 cells. Experiments using freeze-thawed samples were performed twice (runs 1 and 2). Although it was reported that reconstitution efficiency of Sendai virus decreases in a mutant virus such as an addition type in which a foreign gene was inserted into genome (Hasan et al., J. Gen. Virol. 78: 2813-2820, 1997), the M deletion type showed a comparable reconstitution efficiency to that of wild type. The M defect type showed a little lower efficiency. Freeze-thawing of the transfected cells did not significantly affect the plaque number.

As is evident from Table 2 and Figure 7, plaques that are supposed to be derived from cDNAs of the M deletion type and the M defect type were observed. The size of the plaques was much smaller than that of wild type formed in simultaneous positive control (Figure 7; WT1 and WT2). To further examine the shape of the plaques, they were stained with anti-Sendai virus polyclonal antibody, which was produced by the known method by inoculating rabbits with purified Sendai virus that was inactivated as antigens.

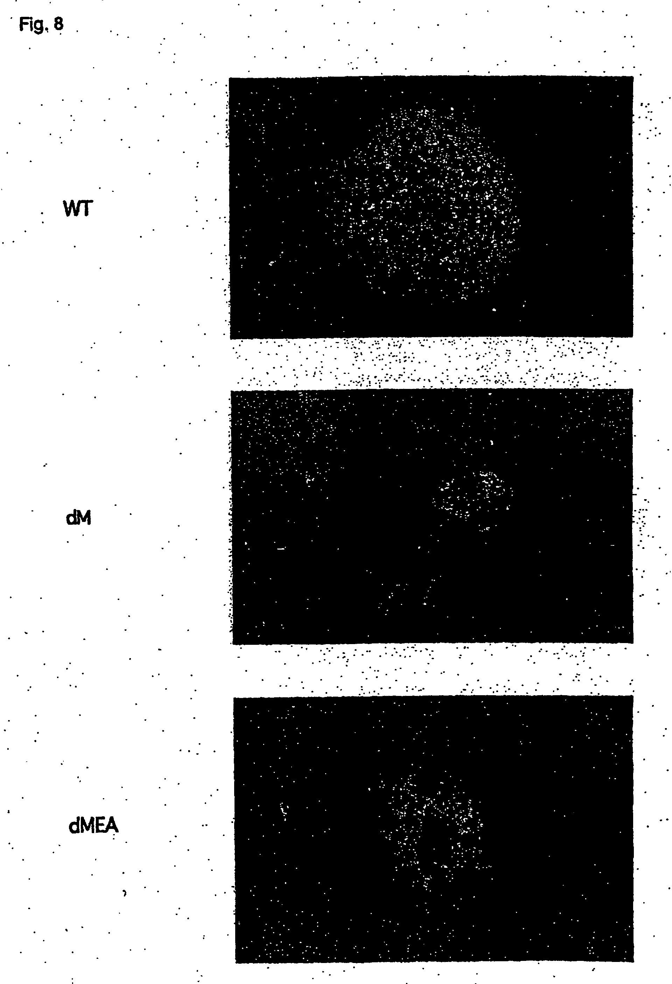

The plaques described in Example 6, which were fixed and stained with Amido Black, were hybridized with anti-Sendai virus antibody, and subsequently with FITC-conjugated anti-rabbit IgG or with anti-mouse IgG antibody, then observed under fluorescence stereoscopic microscope. As a result, signal was detected in plaques of wild type, the M deletion type, and the M defect type, confirming that the plaques were of Sendai viruses (Figure 8).

It was observed that the plaques of wild type cDNA were almost circular, whereas those of the M deletion type and the M defect type cDNAs were small and not circular, having irregular shapes (Figure 8).

### Example 7. Antibody staining of plaques

To determine whether the plaques, different from those of wild type in size and shapes, are derived from cDNA of the M deletion type or the M defect type, the presence of M protein was examined. Namely, these plaques were stained with anti-M protein monoclonal antibody and observed under fluorescence stereoscopic microscope.

Immunofluorescence staining of the plaques was performed as follows.

Plaques were stained with Amido Black. The position of the plaques was examined with the naked eye and marked with a marker pen. Each well of 6-well plates that contains plaques was washed once with 1 ml of PBS, overlaid with 200 µl of 1:40 diluted anti-M protein monoclonal antibody, and incubated at 37°C for 45 minutes in a CO₂ incubator. Then, the well was washed three times with 1 ml of PBS, overlaid with 200 µl of 1:100 diluted FITC-conjugated anti-mouse IgG antibody (Cosmo Bio), and incubated at 37°C for 45 minutes in an incubator. Finally, each well was washed three times with 1 ml of PBS, overlaid with 1 ml of PBS. The staining of the marked plaques was then examined under fluorescence stereoscopic microscope.

Thereafter, the same procedure was repeated processed using anti-Sendai virus polyclonal antibody as the first antibody and FITC-conjugated anti-rabbit IgG antibody (ICN Biomedicals) as the second antibody. The wells were then observed under fluorescence stereoscopic microscope again.

The plaques from wild type cDNA were stained with both anti-Sendai virus antibody and anti-M protein monoclonal antibody. In contrast, plaques from cDNA of the M deletion or defect type were stained with anti-Sendai virus antibody, but not with anti-M protein monoclonal antibody. This indicates that the latter plaques, lacking complete M protein, are derived from cDNA of the M deletion and defect types.

.Accordingly, plaques from Sendai virus gene having a deletion or defect in the M gene were obtained. Since the virus forming plaques does not propagate in chicken eggs, the virus is supposed to be incapable of budding, i.e. not disseminative. In addition, the virus is supposed to be capable of contact infiltration as it forms plaques.

This mode of propagation is probably due to cell fusion mediated by F and HN proteins. Namely, among proteins encoded by Sendai virus, F and HN proteins are expressed as a membrane protein on the cell surface, and M protein supports F and HN proteins as an intracellular anchor in virus budding. In case of the M deletion type and M defect type, budding does not occur because the anchor is lost. However, F and HN proteins are expressed on the cell surface, and cell fusion occurs mediated by these proteins, so that virus genome can be transferred into surrounding cells. In this way, it is possible to realize virus propagation without budding (Figure 9).

### Industrial Applicability

The present invention provides the use of a Sendai virus vector capable of cell infection, autonomous RNA replication, and infiltration through contact, but incapable of dissemination for transferring a foreign gene from a cell to another cell. The use of said Sendai virus vector renders possible a more efficient gene introduction and cell transplantation in gene therapy.

## Claims

1. Use of a Sendai virus RNA in preparing a vector for transferring a foreign gene from a cell to another cell through contact infiltration, wherein the gene encoding Matrix protein is deleted or inactivated in said RNA.

2. Use of a DNA molecule comprising a template DNA for transcribing a Sendai virus RNA in preparing a vector for transferring a foreign gene from a cell to another cell through contact infiltration, wherein the gene encoding Matrix protein is deleted or inactivated in said RNA.

3. Use of a complex comprising a Sendai virus RNA and a virus structure without nucleic acid for transferring a foreign gene from a cell to another cell through contact infiltration, wherein the gene encoding Matrix protein is deleted or inactivated in said RNA.

4. Use of a cell having a complex comprising a Sendai virus RNA and a virus structure without nucleic acid for transferring a foreign gene from said cell to another cell through contact infiltration, wherein the gene encoding Matrix protein is deleted or inactivated in said RNA.

## Patentansprüche

1. Verwendung einer Sendai-Virus-RNS zur Herstellung eines Vektors zum Transferieren eines Fremdgens von einer Zelle zu einer anderen Zelle durch Kontakt-Infiltration, wobei das das Matrix-Protein kodierende Gen in der RNS deletiert oder inaktiviert ist.

2. Verwendung eines DNS-Moleküls, welches umfasst, eine Templat-DNS zum Transkribieren einer Sendai-Virus-RNS zur Herstellung eines Vektors zum Transferieren eines Fremdgens von einer Zelle zu einer anderen Zelle durch Kontakt-Infiltration, wobei das das Matrix-Protein kodierende Gen in der RNS deletiert oder inaktiviert ist.

3. Verwendung eines Komplexes, welcher umfasst, eine Sendai-Virus-RNS und eine Virus-Struktur ohne Nukleinsäure zum Transferieren eines Fremdgens von einer Zelle zu einer anderen Zelle durch Kontakt-Infiltration, wobei das das Matrix-Protein kodierende Gen in der RNS deletiert oder inaktiviert ist.

4. Verwendung einer Zelle mit einem Komplex, welcher umfasst, eine Sendai-Virus-RNS und eine Virus-Struktur ohne Nukleinsäure zum Transferieren eines Fremdgens von der Zelle zu einer anderen Zelle durch Kontakt-Infiltration, wobei das das Matrix-Protein kodierende Gen in der RNS deletiert oder inaktiviert ist.

## Revendications

1. Utilisation d'un ARN de virus Sendai pour la préparation d'un vecteur pour transférer un gène étranger d'une cellule dans une autre cellule par infiltration par contact, ledit gène codant pour protéine Matrice étant délété ou inactivé dans ledit ARN.

2. Utilisation d'une molécule d'ADN comprenant une matrice d'ADN pour transcrire un ARN de virus Sendai pour la préparation d'un vecteur pour transférer un gène étranger d'une cellule dans une autre cellule par infiltration par contact, ledit gène codant pour protéine Matrice étant délété ou inactivé dans ledit ARN.

3. Utilisation d'un complexe comprenant un ARN de virus Sendai et une structure de virus sans acide nucléique pour transférer un gène étranger d'une cellule dans une autre cellule par infiltration par contact, ledit gène codant pour protéine Matrice étant délété ou inactivé dans ledit ARN.

4. Utilisation d'une cellule avec un complexe comprenant un ARN de virus Sendai et une structure de virus sans acide nucléique pour transférer un gène étranger de ladite cellule dans une autre cellule par infiltration par contact, ledit gène codant pour protéine Matrice étant délété ou inactivé dans ledit ARN.


### SEQUENCE LISTING

<110> DNAVEC Research Inc.
<120> RNA virus vector with contact infiltration capability
<130> 50480
<140> EP 99935124.0
   <141> 1999-08-10
<150> JP 1998-227398
   <151> 1998-08-11
<150> PCT/JP99/04333
   <151> 1999-08-10
<160> 4
<170> PatentIn Ver. 2.1
<210> 1
   <211> 49
   <212> DNA
   <213> Artificial Sequence
<220>
   <223> Description of Artificial Sequence: EcoRV-ApaL1 linker
<400> 1
   ggcgatatct atagattccc taagttctca tagtagatgt gcaccggca 49
<210> 2
   <211> 49
   <212> DNA
   <213> Artificial Sequence
<220>
   <223> Description of Artificial Sequence:EcoRV-ApaL1 linker
<400> 2
   tgccggtgca catctactat gagaacttag ggaatctata gatatcgcc 49
<210> 3
   <211> 30
   <212> DNA
   <213> Artificial Sequence
<220>
   <223> Description of Artificial Sequence: A primer used for generating a stop codon within M gene of SeV
<400> 3
   ttaaaggcct aaaccgatct cagaattacg 30
<210> 4
   <211> 24
   <212> DNA
   <213> Artificial Sequence
<220>
   <223> Description of Artificial Sequence: A primer used for generating a stop codon within M gene of SeV
<400> 4
   tatcattccc tgtctcagcc tgcc 24